# EUROPEAN PATENT APPLICATION

(11) **EP 3 315 146 A1**
(43) Date of publication of application: **02.05.2018**
(21) Application number: 16195718.8
(22) Date of filing: 26.10.2016
(51) Int. Cl.: A61L 27/12, A61L 27/32, A61L 27/38, A61L 27/50, A61K 9/00, A61K 9/08

(54) **BONE BIOACTIVE COMPOSITION AND USES THEREOF**

(71) Applicant: Universitat Internacional De Catalunya, Fundació Privada, 08017 Barcelona (ES); Universidad Católica de Murcia, 30107 Guadalupe (Murcia) (ES)
(72) Inventor: Al Atari Abouasi, Maher, 08009 Barcelona (ES); Calvo Guirado, José Luis, 30107 Guadalupe (Murcia) (ES)
(74) Representative: ZBM Patents ApS

(57) **Abstract**

A bone bioactive composition and kits comprising the composition, means for applying it and/or a metal implant are provided. The bone bioactive composition comprises a water-based salt solution comprising disodium hydrogen phosphate and sodium chloride, and may also comprise additional elements. The composition and the kits are useful for promoting osteogenesis, particularly when a metal implant is used but also in case of periodontal diseases.

## Description

### FIELD OF THE INVENTION

The present invention refers to the field of biomedicine and particularly, to compositions for use in promoting osteogenesis. Specifically, the present invention includes compositions for regeneration of dental bone tissue when an implant is used and/or in case of periodontal diseases.

### BACKGROUND ART

One of the major problems that the field of implants faces is the failure of the appropriate engraftment of the implant in the bone, leading to the loss of the implant. In order to reduce implant failure, several modifications of specific surface properties such as structure, chemistry, surface charge and wettability have been investigated to improve protein binding and osteointegration of titanium implants (Albrektsson T, et al. "Osseointegrated titanium implants Requirements for ensuring a long-lasting, direct bone-to-implant anchorage in man" Acta Orthop Scand 1981,52:155-170).

Some peptides and proteins, including collagen and fibronectin, have been used to improve the bioactivity of materials. Both proteins are part of extracellular matrix proteins and have been proven to promote cell adhesion to substrates (Mohan S, et al. "Bone growth factors" Clin Orthop Relat Res 1991,263:30-48). For most cell types, including connective tissue cells such as osteoblasts and their precursors, adhesion to extracellular matrix is essential for survival (Urist MR, et al. "Bone cell differentiation and growth factors" Science 1983,220:680-686; Wozney JM, et al. "Growth factors influencing bone development" J Cell Sci Suppl 1990,13:149-156; Anselme K "Osteoblast adhesion on biomaterials" Biomaterials 2000,21:667).

In addition, it was previously demonstrated that the negative net charge of the alloy's surface oxide enhanced the protein's intrinsic capacity to bind to osteogenic cell integrin receptors and promote cell spreading (Anselme K "Osteoblast adhesion on biomaterials" Biomaterials 2000,21:667; Scotchford CA, et al. "Chemically patterned, metal-oxide-based surfaces produced by photolithographic techniques for studying protein- and cell-interactions. II: Protein adsorption and early cell interactions" Biomaterials 2003,24:1147; Steele JG, et al. "Attachment of human bone cells to tissue culture polystyrene and to unmodified polystyrene: the effect of surface chemistry upon initial cell attachment" J Biomater Sci Polym Ed 1993,5:245; Howlett CR, et al. "Mechanism of initial attachment of cells derived from human bone to commonly used prosthetic materials during cell culture" Biomaterials 1994,15:213; Kilpadi KL, et al. "Hydroxylapatite binds more serum proteins, purified integrins, and osteoblast precursor cells than titanium or steel" J Biomed Mater Res 2001,57:258; García AJ "Get a grip: integrins in cell-biomaterial interactions" Biomaterials 2005,26:7525).

In addition, another major problem faced in odontology is the treatment and prevention of peri-implantitis and periodontitis. Peri-implantitis is defined as a destructive inflammatory process affecting the soft and hard tissues surrounding dental implants produced or induced by bacteria colonizing dental implants and their surroundings. On its side, periodontitis is characterized by the destruction of connective tissue and dental bone support following an inflammatory host response secondary to infection by periodontal bacteria.

Both diseases are currently treated with antibiotics and with increased or improved dental hygiene. Nevertheless, they still lead sometimes to the loss of the implant or the tooth and/or severe damages in the surrounding tissue (connective tissue and dental bone).

Hence, there remains the need to provide means to avoid implant failure, and thus, promoting a properly engraftment of the implant, more specifically, with dental implants which are commonly titanium implants.

In addition, as stated above, there also remains the need to find compositions for use in the treatment or prevention of the periodontal diseases with loss of bone tissue such as periodontitis and peri-implantitis.

### SUMMARY OF THE INVENTION

One problem to be solved by the present invention may be seen as related to the provision of means capable of avoiding implant failure and promoting properly engraftment of the implant with the surrounding tissues, specifically when dental implants are used.

The solution is based on the provision of compositions and kits that have been surprisingly found that promote osteogenesis. Specifically, it promotes, increases, and accelerates the osteogenesis of the surrounding bone tissue of the implant, thus, regenerating or remodeling bone tissue, promoting implant engraftment and avoiding implant loss.

Since the compositions and kits of the invention promote osteogenesis, they are also useful in the treatment of periodontal diseases with loss of bone tissue such as periodontitis and peri-implantitis.

Accordingly, a first aspect of the invention relates to a bone bioactive composition comprising a water-based salt solution comprising disodium hydrogen phosphate and sodium chloride, for use in promoting osteogenesis.

The term "bone bioactive composition" means that the composition is active or reactive with the bone tissue; i.e. that has an effect on, interacts with, or elicits a response from the bone tissue. In other words, that means that the composition interacts and reacts with the bone tissue in some extension. The maximum extension of this "bioactivity" or reaction would be the bone repair. The term "bioactive" is usually used in the field of implants and prosthetics; for example, "bioactive glasses" are a group of surface reactive glass-ceramic biomaterials. They are biocompatible and bioactive, thus being useful as implant materials in the human body to repair and replace diseased or damaged bone.

The term "promoting osteogenesis" has the meaning generally understood in the art; i.e. promoting the development and formation of bone. Osteogenesis is also known as ossification and is the process of laying down new bone material by cells called osteoblasts.

Another aspect of the invention relates to a bone bioactive composition comprising a water-based salt solution comprising 10 mM sodium dihydrogen phosphate, 137 mM sodium chloride, 1.8 mM potassium hydrogen phosphate, 2.7 mM potassium chloride, 8.4 mM magnesium chloride, 10.81 mM calcium chloride, and 68.4 mM EDTA.

Another aspect of the invention is a kit comprising a composition according to the first aspect and a metal implant.

Another aspect of the invention is a kit comprising a composition according to the first aspect and means to apply the composition to the area to be treated.

Another aspect of the invention is the use of the kits mentioned above in promoting osteogenesis.

Another aspect of the invention is a process for preparing the composition according to the first aspect comprising mixing all the elements in solid form of the composition with water until complete homogenization.

Another aspect of the invention relates to the use of a bone bioactive composition, according to the first aspect, for treating the surface of a metal implant prior implantation.

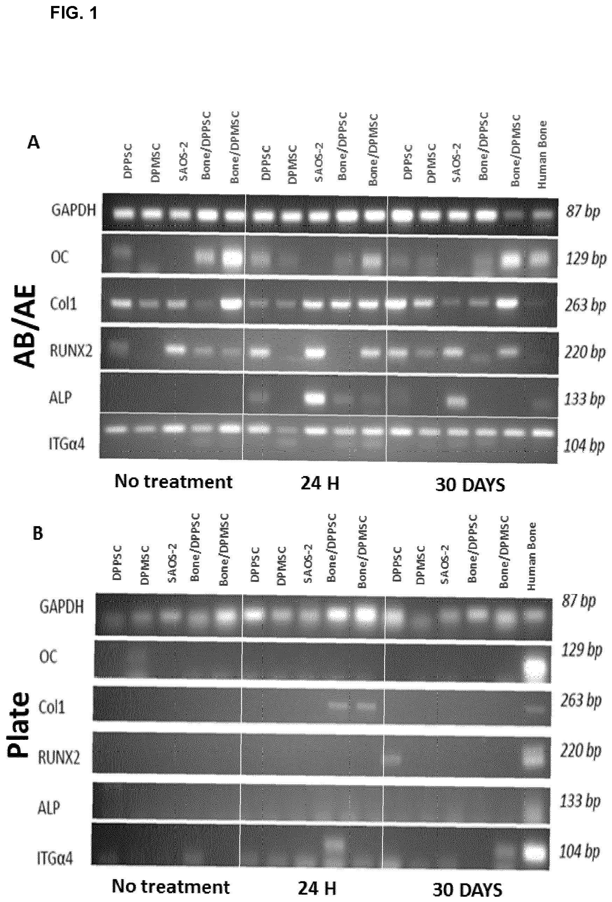

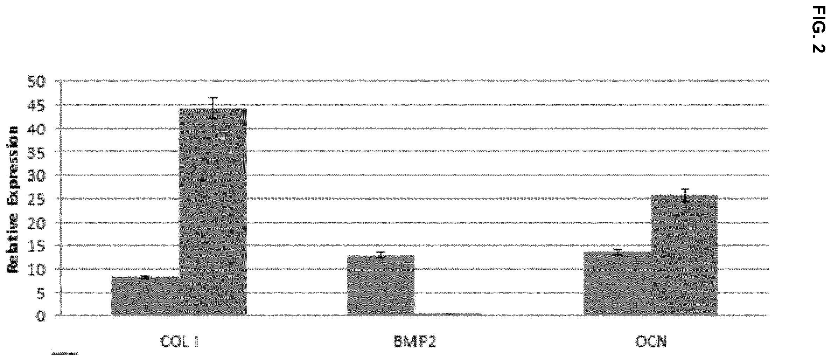

It is believed that the present invention provides the first liquid composition that promotes osteogenesis, with a very easy application and particularly useful when a metal implant is used. Therefore, with the composition of the invention it is not needed to modify the implant surface in order to improve protein binding and osteointegration of implants; which are the strategies currently used in the art. Without being limited to theory, it is believed that the compositions herein proposed promote osteogenesis, favoring bone formation, increasing the production of bone tissue and also improving the quality of the bone produced. This is observed in Example 1 provided below, where influence of the bone bioactive composition in the expression of bone markers in in vitro cell cultures is studied. The different expression of genes shown in FIGs 1 and 2 is correlated with the favoring of bone formation. In addition, when the implant is treated with the bone bioactive composition of the present invention, the production of bone is increased and the quality of the produced bone is higher.

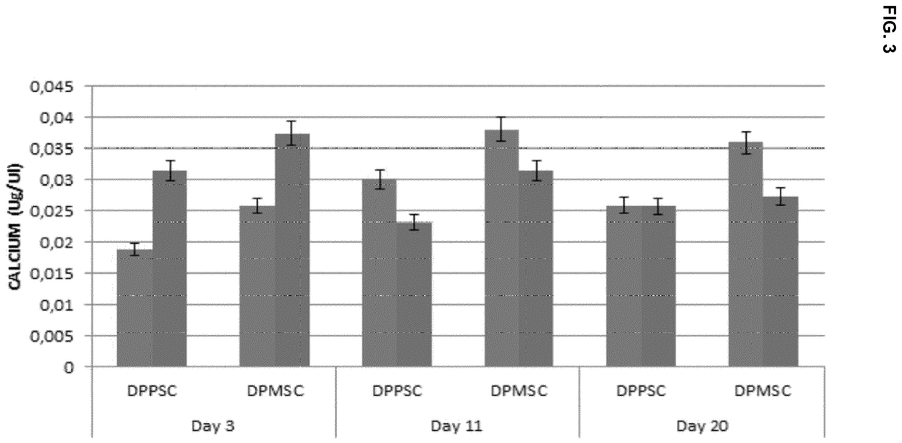

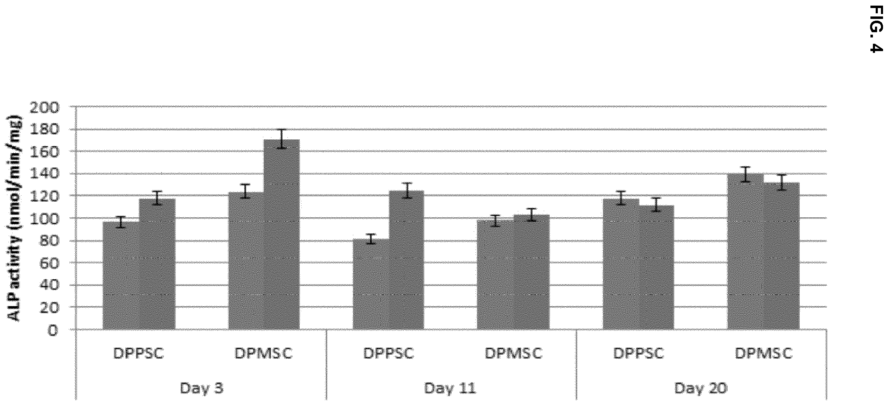

As a second advantageous point, the bone bioactive composition greatly accelerates the process of bone formation. This finding is confirmed with the study in Example 2 and FIG. 3 of the influence of the bone bioactive composition in the calcium excretion and Alcaline Phosphatase (ALP) activity during bone formation in in vitro cell cultures. It can be seen that in the case of cell cultures with implant discs treated with the bone bioactive composition, the peak of calcium excretion can be seen earlier (see increase in day 3), showing an earlier formation of the bone (FIG. 3). Regarding ALP, an increase in the activity of said protein is also related with bone formation. Results obtained for ALP activity appear summarized in FIG. 4. A similar trend as the one seen in FIG. 3 can be seen in FIG. 4, i.e. early peak on day 3 of ALP activity showing early bone formation. Therefore, results obtained for calcium concentration and ALP activity perfectly correlate and are consistent with an early bone formation induced by the bone bioactive composition of the present invention.

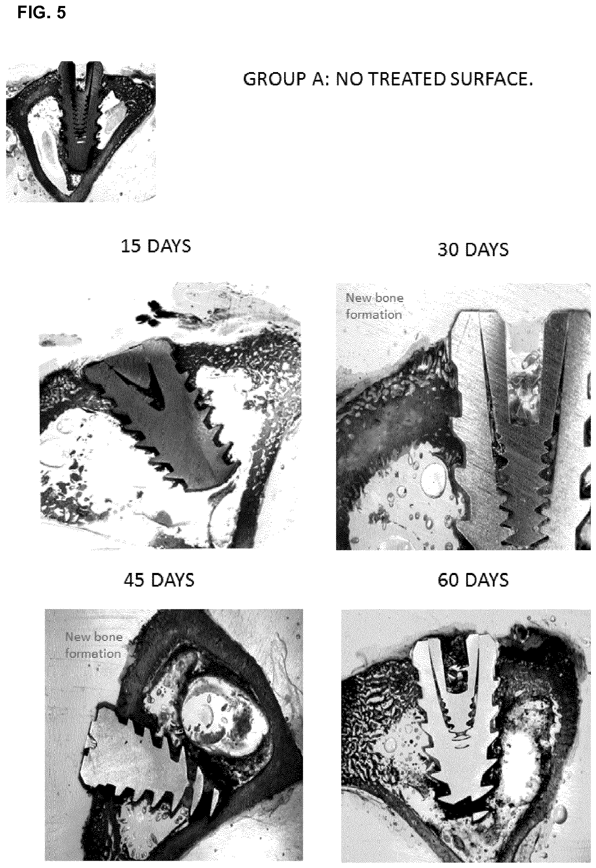

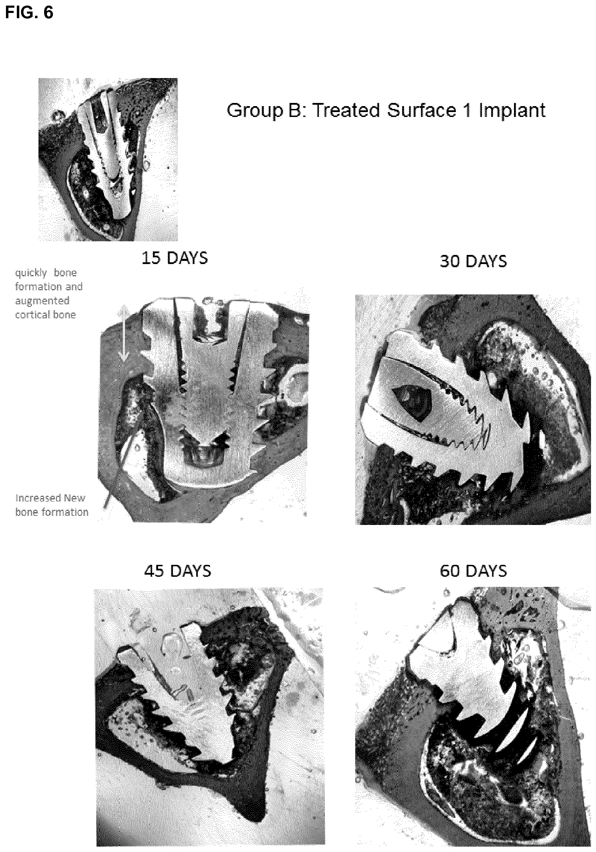

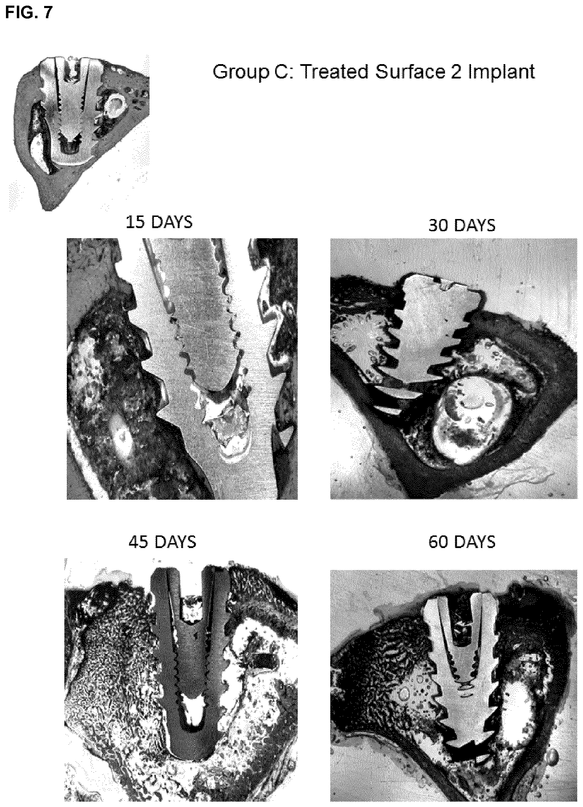

These observations are confirmed in vivo when studying the influence of the bone bioactive composition in the implant-bone engraftment in rabbits (Example 3). For this study 60 dental implant were used divided into three groups: A implant without treatment; B implant+bone active composition at pH 7.4; and C implant+bone active composition at pH 7.6. In general terms, from FIGs. 5 to 7 it can be seen that an earlier formation of bone is induced in Groups B and C (even earlier and greater in the latter). This difference was maintained all throughout the experimental time frame (i.e., until day 60). In addition, from table 3 it is also derivable an earlier and increased BIC (Bone-to-implant Contact) in groups B and C compared to group A. In addition, and surprisingly, as also seen in FIGs 5 to 7, group C showed also an increase in all time points when compared with group B.

Thus, remarkably, without the compositions and kits of the invention a dental implant takes three months to properly engraft, while using the compositions and kits of the invention, engraftment is greatly accelerated and only takes 15 days. This represents a great advantage for the patient and the doctor.

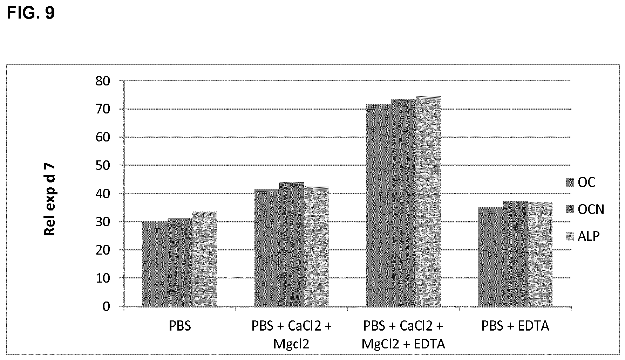

It has been observed in the experiments herein provided (e.g. Example 4 and FIG. 9) that different compositions described above are useful in promoting osteogenesis. Specifically FIG. 9 shows that basic PBS composition enhances bone growth. Two different commercial PBS were additionally tested and all of them gave similar results. Commercial PBS, which contains low concentrations of CaCl₂+MgCl₂, also enhances bone growth. Additionally, the combination PBS+EDTA enhances bone growth in a similar extension that PBS. Nevertheless, remarkably, the combination PBS+CaCl₂+MgCl₂ with higher concentrations of CaCl₂+MgCl₂ presents more osteogenic capacity in comparison with treatment with only PBS or PBS+EDTA and the combination of PBS+CaCl₂+MgCl₂+EDTA shows and even more surprising osteogenic capacity compared to the other treatments.

The compositions of the invention also show relevant advantages from a practical point of view. All the elements of the bone bioactive composition are used in GMP condition; the composition can be used to any kind of titanium surface; its manufacture is not convoluted and is not expensive; the procedure is very easy because the implant is submerged only 2 minutes in the bone bioactive composition prior to insertion into bone, thus, the bone bioactive composition handling is effortless in surgical procedures for bone regeneration or peri-implantitis.

The detailed description and examples shown below are presented for the purposes of providing those skilled in the art with a sufficiently clear and complete explanation of this invention, but should not be considered limitations on the essential aspects contemplated therein, as presented in earlier sections of this description.

### DETAILED DESCRIPTION OF THE INVENTION

### Composition comprising water-based salt solution and preparation thereof

The invention provides a bone bioactive composition comprising a water-based salt solution comprising disodium hydrogen phosphate and sodium chloride, is useful in for use in promoting osteogenesis.

In a particular embodiment, disodium hydrogen phosphate is in a concentration from 8 to 12 mM in the solution and sodium chloride is in a concentration from 130 to 140 mM in the solution. Particularly, disodium hydrogen phosphate is in a concentration from 9 to 11 mM in the solution.

Preferably, the concentration of disodium hydrogen phosphate is in a value or in a subrange between the range 8 to 12 mM, that is, at 8 mM, 8.5 mM, 9 mM, 9.5 mM, 10 mM, 10.5 mM, 11 mM, 11.5 mM or 12 mM. More preferably, the concentration of disodium hydrogen phosphate is 10 mM and more preferably, 10.14 mM.

Independently, in a particular embodiment, sodium chloride is in a concentration from 130 to 140 mM in the solution. Particularly, sodium chloride is in a concentration from 132 to 138 mM in the solution. Preferably, the concentration of sodium chloride is in a value or in a subrange between the range 130 to 140 mM, that is, at 130 mM, 130.5 mM, 131 mM, 131.5 mM, 132 mM, 132.5 mM, 133 mM, 133.5 mM, 134 mM, 134.5 mM, 135 mM, 135.5 mM, 136 mM, 136.5 mM, 137 mM, 137.5 mM, 138 mM, 138.5 mM, 139 mM, 139.5 mM or 140 mM. More preferably, the concentration of sodium chloride is 137 mM, and more exactly, 136.99 mM.

In a particular embodiment, the water-based salt solution further comprises potassium hydrogen phosphate or potassium chloride.

In another particular embodiment, the water-based salt solution further comprises potassium hydrogen phosphate and potassium chloride.

In a more particular embodiment, potassium hydrogen phosphate is in a concentration from 1.4 to 2.2 mM in the solution. Particularly, potassium hydrogen phosphate is in a concentration from 1.6 to 2 mM in the solution. Preferably, the concentration of potassium hydrogen phosphate is in a value or in a subrange between the range 1.4 to 2.2 mM, that is, at 1.4 mM, 1.5 mM, 1.6 mM, 1.7 mM, 1.8 mM, 1.9 mM, 2.0 mM, 2.1 mM or 2.2. mM. More preferably, the concentration of potassium hydrogen phosphate is 1.8 mM and more preferably, 1.76 mM.

In another particular embodiment, potassium chloride is in a concentration from 2.3 to 3.1 mM in the solution. More particularly, potassium chloride is in a concentration from 2.5 to 2.9 mM in the solution. Preferably, the concentration of potassium chloride is in a value or in a subrange between the range 2.3 to 3.1 mM, that is, at 2.3 mM, 2.4 mM, 2.5 mM, 2.6 mM, 2.7 mM, 2.8 mM, 2.9 mM, 3.0 mM, or 3.1 mM. More preferably, the concentration of potassium chloride is 2.7 mM, and more exactly, 2.68 mM.

In a particular embodiment, the pH of the solution is between 7.0 and 8.0. More particularly, the pH is between 7.0 and 7.8. Particularly, the pH is in a value or in a subrange between the range 7.0 and 8.0, that is at 7.0, 7.1, 7.2, 7.3, 7.4, 7.5, 7.6, 7.7, 7.8, 7.9 or 8.0. More preferably, the pH is between 7.4 and 7.8. More preferably, the pH is between 7.5 and 7.7. More preferably, the pH of the solution is 7.6. It is noteworthy, that, surprisingly, the composition of the present invention at pH 7.6 shows even increased effects, as it will be apparent from the examples included below. More specifically, the composition of the present invention, when used at said pH showed an even earlier bone formation and an increased bone formation.

In a particular embodiment, the water-based salt solution further comprises magnesium chloride and calcium chloride. Alternatively, the solution can comprise one salt of magnesium and one salt of calcium with a counterion different but equivalent to chloride, that does not interfere in the solution. In a particular embodiment, magnesium chloride is in a concentration from 2 to 12 mM in the solution and calcium chloride is in a concentration from 7 to 15 mM in the solution. Preferably, magnesium chloride is in a concentration from 4 to 10 mM. Preferably, the concentration of magnesium chloride is in a value or in a subrange between the range 2 to 12 mM, that is, at 2.0 mM, 2.5 mM, 3.0 mM, 3.5 mM, 4.0 mM, 4.5 mM, 5.0 mM, 5.5 mM, 6.0 mM, 6.5 mM, 7.0 mM, 7.5 mM, 8.0 mM, 8.5 mM, 9.0 mM, 9.5 mM, 10.0 mM, 10.5 mM, 11.0 mM, 11.5 mM or 12.0 mM. More preferably, the concentration of magnesium chloride in the solution is 8.4 mM.

Independently, in a particular embodiment, calcium chloride is in a concentration from 7 to 15 mM in the solution. Particularly, calcium chloride is in a concentration from 9 to 12 mM in the solution. Preferably, the concentration of calcium chloride is in a value or in a subrange between the range 7 to 15 mM, that is, at 7.0 mM, 7.5 mM, 8.0 mM, 8.5 mM, 9.0 mM, 9.5 mM, 10.0 mM, 10.5 mM, 11.0 mM, 11.5 mM, 12.0 mM, 12.5 mM, 13.0 mM, 13.5 mM, 14.0 mM, 14.5 mM or 15.0 mM. More preferably, the concentration of calcium chloride is 10.81 mM.

In a particular embodiment, the water-based salt solution further comprises a chelating agent. In a more particular embodiment, the chelating agent is EDTA. In a particular embodiment, the concentration of EDTA in the composition is from 25 mM to 100 mM. Particularly, EDTA is in a concentration from 40 to 80 mM in the solution. Preferably, the concentration of EDTA is in a value or in a subrange between the range 25 mM to 100 mM, and preferably at 68.44 mM.

In a particular embodiment, the bone bioactive composition for promoting osteogenesis comprises a water-based salt solution comprising disodium hydrogen phosphate, sodium chloride, potassium hydrogen phosphate, potassium chloride, magnesium chloride, calcium chloride, and EDTA. In a particular embodiment, the composition comprises a water-based salt solution comprising a water-based salt solution comprising 10 mM sodium dihydrogen phosphate, 137 mM sodium chloride, 1.8 mM potassium hydrogen phosphate, 2.7 mM potassium chloride, 8.4 mM magnesium chloride, 10.8 mM calcium chloride and 68.4 mM EDTA. In a more particular embodiment, the composition comprises a water-based salt solution comprising NaCl 136.99 mM, KCl 2.68 mM, Na₂HPO₄ 10.14 mM, KH₂PO₄ 1.76 mM, MgCl₂ 8.4 mM, CaCl₂ 10.81 mM and EDTA 68.44 mM, at a pH of between 7.0 and 7.8, and more preferably of 7.6.

Thus, a bone bioactive composition particularly useful for the purposes of the invention comprises a water-based salt solution comprising 10 mM sodium dihydrogen phosphate, 137 mM sodium chloride, 1.8 mM potassium hydrogen phosphate, 2.7 mM potassium chloride, 8.4 mM magnesium chloride, 10.8 mM calcium chloride and 68.4 mM EDTA. In a particular embodiment the pH of the solution is 7.6.

Thus, the examples provided demonstrate that different compositions are useful in the promotion of osteogenesis for the purposes of the invention. These include particularly, compositions based only on disodium hydrogen phosphate and sodium chloride; compositions further comprising potassium hydrogen phosphate and/or potassium chloride; compositions further comprising magnesium and calcium chloride, and compositions further comprising EDTA.

Some examples of commercially available solutions are described herein as examples of compositions that can also be used for the purposes of the invention. The water-based salt solution used in the invention is often referred as "Phosphate Buffered Saline" (abbreviated PBS). PBS refers to a buffer solution containing at least one phosphate and which is commonly used to perform molecular biology techniques and in cell culture, to perform washes and/or to prepare reagents. The basic components are disodium hydrogen phosphate and sodium chloride and, in some formulations, potassium chloride and potassium dihydrogen phosphate. The osmolarity and ion concentrations of the solutions match those of the human body (isotonic). PSB can be stored at room temperature.

There are many different ways to prepare PBS solutions. Some formulations do not contain potassium and magnesium, while other ones contain calcium and/or magnesium. Some examples of PBS are described herein.

**Table A. The most common composition of PBS (1X)**

| Salt | Concentration (mmol/L) | Concentration (g/L) |
|---|---|---|
| NaCl | 137 | 8.0 |
| KCl | 2.7 | 0.2 |
| Na₂HPO₄ | 10 | 1.42 |
| KH₂PO₄ | 1.8 | 0.24 |

Method of preparation:_Start with 800 mL of distilled water to dissolve all salts. Adjust the pH to 7.4 with HCl. Add distilled water to a total volume of 1 liter. The resultant 1x PBS should have a final concentration of 10 mM PO₄³⁻, 137 mM NaCl, and 2.7 mM KCI.

**Table B. Cold Spring Harbor Protocol:**

| reagent | MW | mass (g) 10X | [M] 10X | mass (g) 1X | [M] 1X | [mM] 1x |
|---|---|---|---|---|---|---|
| Na₂HPO₄ | 141.95897 | 14.1960 | 0.1000 | 1.41960 | 0.0100 | 10.0 |
| KH₂PO₄ | 136.08569 | 2.4496 | 0.0180 | 0.24496 | 0.0018 | 1.8 |
| NaCl | 58.44300 | 80.0669 | 1.3700 | 8.00669 | 0.1370 | 137.0 |
| KCl | 74.55150 | 2.0129 | 0.0270 | 0.20129 | 0.0027 | 2.7 |
| pH = 7.4 | | | | | | |

The pH of the 10x stock solution of PBS is ∼6.8, but when diluted with water to 1x PBS it should change to 7.4. When making buffer solutions, it is good practice to always measure the pH directly using a pH meter. If necessary, pH can be adjusted using hydrochloric acid or sodium hydroxide.

The simplest way to prepare a PBS solution is to use PBS buffer tablets or pouches. They are formulated to give a ready-to-use PBS solution upon dissolution in a specified quantity of distilled water. They are available in the standard volumes: 100, 200, 500 and 1000 mL, and 10, 25, 50 and 100 L.

PBS can be stored at room temperature or in the refrigerator. However, concentrated stock solutions may precipitate when cooled and should be kept at room temperature until precipitate has completely dissolved before use.

**Table C. Phosphate-buffered saline (PBS) (Cold Spring Harbor)**

| Reagent | Amount to add (for 1× solution) | Final concentration (1 x) | Amount to add (for 10x stock) | Final concentration (10x) |
|---|---|---|---|---|
| NaCl | 8 g | 137 mM | 80 g | 1.37 M |
| KCl | 0.2 g | 2.7 mM | 2g | 27 mM |
| Na₂HPO₄ | 1.44 g | 10 mM | 14.4 g | 100 mM |
| KH₂PO₄ | 0.24 g | 1.8 mM | 2.4 g | 18 mM |
| If necessary, PBS may be supplemented with the following: | | | | |

| Reagent | Amount to add (for 1× solution) | Final concentration (1×) | Amount to add (for 10× stock) | Final concentration (10×) |
|---|---|---|---|---|
| CaCl₂•2H₂O | 0.133 g | 1 mM | 1.33 g | 10 mM |
| MgCl₂•6H₂O | 0.10 g | 0.5 mM | 1.0 g | 5 mM |

PBS can be made as a 1 × solution or as a 10× stock. To prepare 1 L of either 1 × or 10× PBS, dissolve the reagents listed above in 800 mL of H₂O. Adjust the pH to 7.4 (or 7.2, if required) with HCl, and then add H₂O to 1 L. Dispense the solution into aliquots and sterilize them by autoclaving for 20 min at 15 psi (1.05 kg/cm²) on liquid cycle or by filter sterilization. Store PBS at room temperature.

For a 10 liter stock of 10x PBS can be prepared by dissolving:
800 g NaCl,
20 g KCI,
144 g Na₂HPO₄ · 2H₂O
24 g KH₂PO₄
8 L of distilled water.
After complete mixing, top up final solution to 10 L. The pH of the 10X stock will be approximately 6.8, but when diluted to 1x PBS it should change to 7.4. When making buffer solutions, it is good practice to always measure the pH directly using a pH meter. If necessary, pH can be adjusted using hydrochloric acid or sodium hydroxide.

On dilution, the resultant 1x PBS should have a final concentration of 137 mM NaCl, 10 mM Phosphate, 2.7 mM KCI, and a pH of 7.4.

pH is adjusted with an acid or a base, for instance with hydrochloric acid or phosphoric acid and sodium hydroxide.

PBS is also referred as PBS 10 mM because the concentration of Na₂HPO₄ is 10 mM (PBS 1x 1 L).
Dulbecco's Phosphate Buffered Saline (DPBS): There is no significant difference between PBS and DPBS, unless DPBS has less phosphate concentration; the concentration of Na₂HPO₄ is 10 mM in PBS and 8.1 mM in DPBS and the concentration of KH₂PO₄ is 1.8 mM in PBS and 1.41 mM in DPBS.

### Product forms

The composition according to the first aspect of the invention is usually in liquid form ready to be used; therefore it can be commercially sold as liquid in a suitable container, in single doses (e.g. of 1 ml, 2 ml, 3 ml, 5 ml, 10 ml or higher, or in a higher volume (e.g. 25 ml, 50 ml, 100 ml, 1 liter, 10 liters).

The composition can also adopt suitable forms to make the application easier, for example in the form of gel to be applied in the area to be treated or in the tissues surrounding the implant.

Thus, in particular embodiments, the composition according to the first aspect is in liquid form, in the form of a gel, a varnish or a spray.

In a particular embodiment, the elements of the composition are in solid form, with all the elements of the composition together in a single container or in different containers (e.g. a vial) or in the form of solid tablet, in both cases, to be reconstituted just before use. To this end, the commercial product can comprise the elements of the composition in a container/containers or in the form of tablet, and a volume of water in a container, perfectly measured to be mixed with the solid elements, thus achieving the final solution concentrations specified in other sections of this description. Additionally, water can be acidified or basified in order to obtained the desired pH when mixing with the elements of the composition.

The skilled person will routinely select the amounts of the elements of the composition to obtain a final composition comprising a water-based salt solution depending on the final volumes of composition.

The term "container" herein is used to denote any recipient, having a closure that is suitable for packaging a unit dosage amount of a solid or liquid composition. It will be understood that equivalent forms of packaging, such as a vial, an ampoule, a disposable syringe, a syringe cartridge or a pre-filled syringe, are encompassed by this embodiment of the invention. The containers should be made of a material to preserve stability of the elements of the composition for preferably storing it at room temperature, but also refrigerated.

An aspect of the invention as said above is a kit comprising a composition according to the first aspect and a metal implant. The metal implant or the metal prosthesis can be anyone used in odontology and traumatology. In a particular embodiment, the metal implant is a dental implant. Dental implants are usually made of Titanium to very specific and accurate tolerances. The most important Titanium alloys for clinical applications are Ti-6AI-4V, Ti-3AI-2.5V and Ti-6AI-7Nb. Dental implants are also made of monolithic Zirconium-oxide. Particularly, the dental metal implant is a dental titanium implant.

In a particular embodiment, the kit comprises the implant and the composition of the invention in separate parts. However, in a particular embodiment, the commercial presentation is the implant submerged into the liquid composition in a container. In a particular embodiment, the kit further comprises means to apply the composition; said means are explained hereinafter.

The proposal of the invention can also be useful to promote bone regeneration of areas that are not in contact with an implant but with presence of bone loss. Examples of the above are in case of periodontal diseases. In some cases, the article of manufacture is only the composition in liquid form or in solid form, because the professional has at his disposal the other elements to prepare the composition and to use and apply the composition to the patient (e.g. water and means for application). Nevertheless, another aspect of the invention is a kit comprising a composition according to the first aspect and means to apply the composition to the area to be treated. Particularly, the means to apply the composition are a syringe, a cotton rod and a textile gauze. In a particular embodiment, the means are to apply the composition to the buccal cavity.

Preferred means to apply the composition in case of peri-implantitis or periodontitis (i.e. that an application to the bone pocket is needed) are a mouthguard, also known as occlusal splints, mouthprotector, mouth piece, gumshield, gumguard, nightguard, bite or mouth splint, or bite plane. This is a removable dental appliance to fit the upper or lower arches of teeth.

In another embodiment, also in order to facilitate the use, the kit further comprises means for adding water into the container with the composition in solid form. The means are preferably a syringe equipped with a disposable perforating needle that is used for drawing water from its container and putting into the container with the composition in solid form.

Another aspect of the invention is the use of the kits described above for use in promoting osteogenesis.

Besides the elements cited in this description conforming the water-based solution according to the first aspect, the composition of the invention can comprise additional elements, carriers or excipients, adequate to have a biocompatible composition. These products can adopt the form of a pharmaceutical product, a medicament, a food supplement or an oral care product.

The skilled in the art will use other forms of presentation appropriate to the composition of the invention, normally used in the pharmaceutical and chemical reagents industries.

### Applications of the compositions and the kits

As discussed above, a first aspect of the invention relates to a bone bioactive composition as described above, for use in promoting osteogenesis. This aspect can be alternatively formulated as the use of any of the compositions of the invention for the manufacture of a pharmaceutical product, a medicament, a food supplement or an oral care product for promoting osteogenesis. This aspect may be also alternatively formulated as a method for promoting osteogenesis comprising administering to the subject in need thereof an effective amount of any of the compositions of the invention. Alternatively, it can also be formulated as the use of a bone bioactive composition as described above, in promoting osteogenesis. The same aspect can also be formulated as a method of promoting osteogenesis by using a bone bioactive composition as described above.

Thus, the invention provides a bone bioactive composition for use a medicament.

In a particular embodiment, the bone bioactive composition is used in the promotion of osteogenesis when a metal implant is used. In a particular embodiment, the metal implant is a dental titanium implant. The use of the composition or the kits of the present invention favor an early engraftment of the metal implant, inducing the earlier formation of the bone around the implant and, also increasing the quantity of bone formed. In addition, the bone formed is of an increased quality. All the above features contribute to an earlier and stronger engraftment of the implant as well as to a reduced implant loss. Without the compositions and kits of the invention a dental implant takes three months to properly engraft, while using the compositions and kits of the invention, engraftment is greatly accelerated and only takes 15 days. This represents a great advantage for the patient and the doctor.

Hence, the compositions or kits of the present invention are used to improve the engraftment of metal implants (preferably dental titanium implants).

In addition, since the composition of the present invention induces early and/or increased ossification it has also been found useful in the treatment of periodontal diseases such as periodontitis and peri-implantitis, two diseases associated with bacterial infections affecting the connective tissue and the dental bone, leading to the destruction thereof and the loss of implants or teeth, respectively. Said composition, due to its capacity for early and accelerated and increased bone generation is also useful for promoting osteogenesis in the treatment of a periodontal disease, such as peri-implantitis and periodontitis. This may be alternatively formulated as a method for the prevention and/or treatment of a periodontal disease, comprising administering to the subject in need thereof an effective amount of any of the compositions of the invention.

Another aspect of the invention relates to the use of a bone bioactive composition, according to the first aspect, for treating the surface of a metal implant prior implantation.

The skilled person will select the appropriate volumes of composition according to the surface and condition to be treated. In a particular embodiment, the composition is applied to the area until the bleeding of said area that means that the stem cells have emerged. In a particular embodiment, some milliliters of composition are applied to the area.

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein. The following examples and drawings are provided herein for illustrative purposes, and without intending to be limiting to the present invention.

### BRIEF DESCRIPTION OF THE DRAWINGS

FIG. 1. RT-PCR gene expression analysis of differentiation markers (OC, COL I, RUNX2, ALP and ITGα4) at the second week of cell differentiation. GAPDH was used as a housekeeping gene. A: Titanium disc Ti-6AI-4V (AB/AE) treated with bone bioactive composition. B: Titanium disc Ti-6AI-4V with Calcium phosphate (Plate) treated with bone bioactive composition.
FIG. 2. Q RT-PCR analysis of osteogenic markers (COL I, BMP2 and OCN) of DPPSC at days 21 of cell differentiation. GAPDH was used as a housekeeping gene.
FIG. 3. ALP activity. Calcium activity analysis of DPPSC and DPMSC at days 3,11 and 20 of cell differentiation on titanium discs treated with bone bioactive composition. GAPDH was used as a housekeeping gene.
FIG. 4. ALP activity analysis of DPPSC and DPMSC at days 3, 11 and 20 of cell differentiation on titanium discs treated with bone bioactive composition. GAPDH was used as a housekeeping gene.
FIG: 5. Histomorphometric analysis of experimental rabbits with group A at 15, 30, 45 and 60 days.
FIG. 6. Histomorphometric analysis of experimental rabbits with group B at 15, 30, 45 and 60 days.
FIG. 7. Histomorphometric analysis of experimental rabbits with group C at 15, 30, 45 and 60 days.
FIG. 8. Quantifications of new bone formation of experimental rabbits with group A, B and C at 15, 30, 45 and 60 days.
FIG. 9. Q RT-PCR analysis of osteogenic markers (OC, OCN and ALP) of DPPSC at day 7 of cell differentiation on treated titanium discs. GAPDH was used as a housekeeping gene. Expression related to no treatment discs.

### EXAMPLES

### Example 1. Influence of the bone bioactive composition in the expression of bone markers in in vitro cell cultures.

### Cell isolation:

### 1. Culture in DPPSC medium

Cells were cultured on 75 cm² flask with DPPSC medium containing as base medium 60% Dulbecco modified Eagle's medium (DMEM)-low glucose (Sigma, United States) and 40% MCDB-201 (Sigma, United States) supplemented with 1x insulin-transferrin-selenium (hereinafter, ITS; Sigma, United States), 1x linoleic acid-bovine serum albumin (hereinafter, LA-BSA; Sigma, United States), dexamethasone 10⁻⁹ M (Sigma, United States), 10⁻⁴ M ascorbic acid 2-phosphate (Sigma, United States), 100 units of penicillin 1000 units of streptomycin (PAA, Life Technologies, USA), 2% foetal bovine serum (hereinafter, FBS; Sigma, United States), 10 ng/ml of Human Platelet Derived Growth Factor-BB (hereinafter, hPDGF-BB; R & D Systems, United States) and 10 ng/ml of Epidermal Growth Factor (hereinafter, EGF; R & D Systems, United States). The flasks were previously covered with 10 ml of 100ng/ml fibronectin (Life Technologies, USA) and incubated at 37 °C, 5% CO₂ concentration during 1 hour.

During the two weeks of primary culture, the medium was changed every 3 days and the cells confluence was maintained at 30%, since higher rates of confluence lead to cell maturation and changes in morphology and genotype.

### 2. Culture in DPMSC medium (hereinafter, cells cultured under these conditions are referred as DPMSCs):

Cells were cultured on 75 cm² flask with DPMSC medium containing Dulbecco modified Eagle's medium (DMEM) (Biochrom, United Kingdom) supplemented with 2 ng/ml basic Fibroblast Growth Factor (hereinafter, bFGF) and 10% FBS (Hyclone, USA). The flasks were previously covered with 10 ml of 100 ng/ml fibronectin (Life Technologies, USA) and incubated at 37 °C, 5% CO₂ concentration during 1 hour.

Cells were seeded at 300,000 cells/cm2 density, and the medium was changed every three days. The confluence was settled at 50-80 % in order to maintain cell morphology and multipotency.

### 3. Culture in Saos medium (hereinafter, cells cultured under these conditions are referred as Saos cells):

Subculture P 12 cells were seeded at 500,000 cells/cm² density, and the medium was changed every three days. The confluence was settled at 80% were unfreezed and seeded in 75 cm² flasks with Saos medium containing DMEM (Invitrogen, United States), supplemented with 10 % FBS ([Hyclone, USA]), 2mm L-Glutamine (Sigma USA), 100 u/ml penicillin (Life Technologies, USA), 1000 u/ml streptomycin (Life Technologies, USA), The flasks were previously covered with 10 ml of 100ng/ml fibronectin (Life Technologies, USA) and incubated at 37 °C, 5 % CO₂ concentration during 1 hour.

The medium was changed every three days. The subcultures were done when cells were at 80 % of confluence.

### Preparation of the implants:

To analyze the influence of the bone bioactive composition of the present invention in the interaction between the above-mentioned cell cultures and metal implants (more precisely, dental titanium implants), discs from the implants were prepared and treated as follows:

Titanium discs were obtained by cutting commercially available titanium alloy Ti₆Al₄V (Ti-6AI-4V), also other discs where obtained from other titanium alloys, in particular Ti-6AI-4V Eli y Ti-5AI-2.5Sn. There were no differences in behavior between discs. The discs measured 2.0 mm in thickness and had a diameter of 14.0 mm. The surface of said discs was then alumina-blasted and acid-etched (hereinafter, AB/AE) which proportioned roughness and increased the implant (disc) surface area.

The form of the probes (whether discs, plates or whatever other shape) has no influence on the results.

### Preparation of the bone bioactive composition:

8 g of NaCl, 0.2 g of KCI, 1.44 g of Na₂HPO₄, 0.24 g of KH₂PO₄, 0.80 g of MgCl₂, 1.20 g of CaCl₂ and 20 g of EDTA were dissolved in 800 ml of water. After all the components were correctly dissolved the volume of the solution was brought to 1 l.

Prior to contacting the above-mentioned discs with the cell cultures, the discs were treated with 100 microliters of the bone bioactive composition, incubated at room temperature (37 °C for 1 day). This surface treatment was divided into three groups: surface treatment evaluation at 1 day, 1 month of cell culture and no surface treatment.

### Osteoblast differentiation:

To analyze osteoblast differentiation, the three cell populations mentioned above were cultured, independently, on the titanium discs. Cells were seeded in titanium discs of Ti₆Al₄V discs (treated as mentioned above, i.e., discs treated AB/AE and incubated or not with the bone bioactive composition of the present invention) in 24-well plates at a density of 1x10³ cells per cm². For the different cell types used, the culture conditions for osteoblast differentiation were:
- DPPSCs and DPMSCs: Passage five DPPSCs and DPMSCs from the same clone were used for osseous differentiation with osteogenic medium, which contained; as base medium α-MEM (Gibco) and RPMI, supplemented with 10% FBS, 10 mM β-glycerol phosphate (Sigma, United States), 50 mM L-ascorbic acid (Sigma, United States)), dexamethasone 0.01 mM and 1% solution of penicillin/streptomycin. The suspension was tipped out in 75 cm² flasks including the titanium disks with the different treatments mentioned above. The medium was changed every 3 days.
- Saos cells were cultured under the same conditions except for the medium, which was the same as mentioned above for these cells.

Bearing in mind the above cell culture conditions, the following groups were generated and analyzed to see if there were differences in the expression of several markers of osteodifferentiation, mainly OC, COL I, RUNX-2, ALP and ITGα4. As housekeeping gene, the expression of GAPFH was also analyzed:
- DPPSCs cultured with non-treated (i.e., without the bone bioactive composition) implant disc and under non-differentiating conditions.
- DPMSCs cultured with non-treated (i.e., without the bone bioactive composition) implant disk and under non-differentiating conditions.
- Saos cells with non-treated (i.e., without the bone bioactive composition) implant disc.
- DPPSCs cultured with non-treated (i.e., without the bone bioactive composition) implant disk and under osteoblast differentiating conditions.
- DPMSCs cultured with non-treated (i.e., without the bone bioactive composition) implant disk and under osteoblast differentiating conditions.
- DPPSCs cultured with treated (i.e., with the bone bioactive composition) implant disc and under non-differentiating conditions during 24 hours.
- DPMSCs cultured with treated (i.e., with the bone bioactive composition) implant disk and under non-differentiating conditions during 24 hours.
- Saos cells with treated (i.e., with the bone bioactive composition) implant disc during 24 hours.
- DPPSCs cultured with treated (i.e., with the bone bioactive composition) implant disk and under osteoblast differentiating conditions during 24 hours.
- DPMSCs cultured with treated (i.e., with the bone bioactive composition) implant disk and under osteoblast differentiating conditions during 24 hours.
- DPPSCs cultured with treated (i.e., with the bone bioactive composition) implant disc and under non-differentiating conditions during 30 days.
- DPMSCs cultured with treated (i.e., with the bone bioactive composition) implant disk and under non-differentiating conditions during 30 days.
- Saos cells with treated (i.e., with the bone bioactive composition) implant disc during 30 days.
- DPPSCs cultured with treated (i.e., with the bone bioactive composition) implant disk and under osteoblast differentiating conditions during 30 days.
- DPMSCs cultured with treated (i.e., with the bone bioactive composition) implant disk and under osteoblast differentiating conditions during 30 days.
- Human bone.
- Same groups were also analyzed without implant disk i.e. cultivating the cells with Titanium plate discs covered by calcium phosphate (which causes calcification).

To see the RNA expression of the markers mentioned above, total RNA was extracted at 1 and 30 days of differentiation (and for the controls when was RNA extracted) using Trizol (Invitrogen, United States). The sample was treated with DNAse (Promega, United States) and, RNA was isolated following manufacturer's instructions of UltraCleanTM Tissue & Cells RNA Isolation Kit (MoBio, United States). Two (2) μg of RNA aliquots were treated with DNase I (Invitrogen, United States) and reverse-transcribed using Transcriptor First Strand cDNA Synthesis Kit (Roche, Switzerland). RT-PCR was performed using the primers on the following Table 1 for the amplification of OC, COL I, RUNX-2, ALP, ITGα4, and GAPDH.

**Table 1. Primers used for the amplification of OC, COL I, RUNX-2, ALP, ITGα4, and GAPDH**

| **GENE** | **Accession Number** | **FORWARD PRIMER (5'-3')** | **REVERSE PRIMER (5'-3')** | **PRODUCT SIZE (bp)** | **USE** |
|---|---|---|---|---|---|
| **ALP** | NM_000478 | GGACATGCAGTACGAGCTGA | GTCAATTCTGCCTCCTTCCA | 133 | RT-PCR |
| | | **(SEQ ID NO: 1)** | **(SEQ ID NO: 2)** | | |
| **ALP** | NM_000478 | CCGTGGCAACTCTATCTTTGG | GCCATACAGGATGGCAGTGA | 79 | q RT-PCR |
| | | **(SEQ ID NO: 3)** | **(SEQ ID NO: 4)** | | |
| **COL1** | NM_000088 | ACTGGTGAGACCTGCGTGTA | CAGTCTGCTGGTCCATGTA | 263 | RT-PCR |
| | | **(SEQ ID NO: 5)** | **(SEQ ID NO: 6)** | | |
| **COL1** | NM_000088 | CCCTGGAAAGAATGGAGATGA T | ACTGAAACC TCTGTGTCCCTTCA 1 | 39 | qRT-PCR |
| | | **(SEQ ID NO: 7)** | **(SEQ ID NO: 8)** | | |
| **OC** | NM_199173 | GTGCAGCCTTTGTGTCCAA | GCTCACACACCTCCCTCCT | 129 | RT-PCR |
| | | **(SEQ ID NO:9)** | **(SEQ** ID **NO:10)** | | |
| **OC** | NM_199173 | AAGAGACCCAGGCGCTACC T | AAC TCGTCACAGTCCGGATTG | 110 | q RT-PCR |
| | | **(SEQ ID NO: 11)** | **(SEQ ID NO:12)** | | |
| **RUNX2** | NM_001146038 | TTACTGTCATGGCGGGTAAC | GGTTCCCGAGGTCCATCTA | 220 | RT-PCR |
| | | **(SEQ ID NO: 13)** | **(SEQ ID NO: 14)** | | |
| **RUNX2** | NM_001146038 | AGCAAGGTTCAACGATCTGAG AT | TTTGTGAAGACGGTTATGGTC AA | 81 | q RT-PCR |
| | | **(SEQ** ID **NO: 15)** | **(SEQ ID NO: 16)** | | |
| **ITGα4** | NM_002204 | TCCGAGTCAATGTCCACAGA | GCTGGGCTACCCTATTCCTC | 88 | RT-PCR q RT-PCR |
| | | **(SEQ** ID **NO: 17)** | **(SEQ ID NO: 18)** | | |
| **GAPDH** | NM_002046 | CTGGTAAAGTGGATATTGTTGCC AT | TGGAATCATATTGGAACATGTAA ACC | 81 | RT-PCR q RT-PCR |
| | | **(SEQ ID NO: 19)** | **(SEQ ID NO: 20)** | | |

Said RT-PCR amplifications were run in and agarose gel 2%. Results were visualized under UV Light and are shown in FIG. 1.

In addition, FIG. 2 shows the results of relative expression for the genes COL I, BMP2 and OCN at day 0 and 30.

| | | | |
|---|---|---|---|
| BMP2 (Bone morphogenetic protein) | Forward | GCG GAA ACG CCT TAA GTC CA (SEQ ID NO: 21) | 20 |
| | Reverse | GTG GAG TTC AGA TGA TCA GC (SEQ ID NO: 22) | 20 |
| OCN | Forward | GCAGACCTGACATCCAGTAC (SEQ ID NO: 23) | 57.7 |
| | Reverse | TAATCTGGACTGCTTGTGGC (SEQ ID NO: 24) | 57.7 |

Primers used for COL I were the same identified in Table 1.

The different expression of genes shown in FIGs 1 and 2 is correlated with the favouring of bone formation. In addition, when the implant is treated with the bone bioactive composition of the present invention, the production of bone is increased and the quality of the produced bone is higher.

### Example 2. Influence of the bone bioactive composition in the calcium excretion and Alcaline Phosphatase (hereinafter, ALP) activity during bone formation in in vitro cell cultures.

Calcium concentration in the cell culture medium (i.e., secreted calcium) was measured in DPPSCs and DPMSCs cultured with implant discs treated or not with bone bioactive composition under differentiation conditions, in accordance with what has previously been explained. Said secreted calcium is related with bone formation.

Calcium concentration was measured by analyzing the supernatant of each cell population at days 3, 11 and 20 of differentiation. The analysis was performed using a calcium colorimetric assay kit (BioVision, United States) through a chromogenic complex (λ = 575 nm) formed between calcium ions and 0-cresolphthalein. This analysis provides a wavelength detected at 575 nm and this value determines the calcium concentration.

Results obtained for calcium concentration are summarized in FIG. 3 It can be see that in the case of cell cultures with implant discs treated with the bone bioactive composition, the peak of calcium excretion can be seen earlier (see increase in day 3), showing an earlier formation of the bone.

Regarding ALP, an increase in the activity of said protein is also related with bone formation. Hence, ALP activity was measured in the supernatant of the above-mentioned cell cultures also at days 3, 11 and 20. Said activity was measured using an alkaline phosphatase kit (BioSystems, USA) according to the manufacturer's instructions. The absorbance of each sample was measured at a wavelength of 405 nm at different times (day 3, 11 and 20 during cell differentiation) wherein an increase in the ALP activity is observed in the treated group vs not treated.

Results obtained for ALP activity appear summarized in FIG. 4. A similar trend as the one seen in FIG. 3 can be seen in FIG. 4, i.e. early peak on day 3 of ALP activity showing early bone formation.

Therefore, results obtained for calcium concentration and ALP activity perfectly correlate and are consistent with an early bone formation induced by the bone bioactive composition of the present invention.

### Example 3. Influence of the bone bioactive composition in the implant-bone engraftment in rabbits.

For this study 60 dental implants were used divided into three groups (n=20 per group). Treatment for each of the three study groups appears described in Table 2.

**Table 2. Details of the additional treatment applied to the implants in the different groups of the study.**

| Group | Implant surface treatment |
|---|---|
| A | No additional treatment |
| B | Bone bioactive composition of Example 1, pH adjusted at 7.4 |
| C | Bone bioactive composition of Example 1, pH adjusted at 7.6 |

The implants were treated by blasting with 50-100 µm TiO₂ particles, followed by ultrasonic cleaning with an alkaline solution Riozyme IV-E Neutro Gold (Indústria Farmacêutica Rioquímica Ltda, São José do Rio Preto, Brazil), washing with distilled water and pickling with maleic acid (HO₂CCH₂CHOHCO₂H). For the implants used in group A the above was the only treatment applied. For experimental groups B and C: the implants were treated initially using the above procedure and after with the corresponding bone bioactive composition using 100 µL for 1 h.

Fifteen adult New Zealand rabbits (*Oryctolagus cuniculus*) with approximately 4.5 ± 0.5 kg were used in this study. Four implants were installed per animal (2 per tibia). The animals were sacrificed 15, 30, 45 and 60 days after surgery.

For the histomosphometric analysis, bone blocks of the tibiae, with inserted implants, were removed from each animal, fixed in 10% of formaldehyde solution for 7 days, and dehydrated in increasing ethanol solutions (60%, 70%, 80%, and 99%) for 24-56 h, as previously described (Yang J et al. "Effects of oestrogen deficiency on rat mandibular and tibial microarchitecture" Dentomaxillofac Radiol 2003 Jul,32(4):247-51). Subsequently, the samples were embedded in Technovit 7200 VLC resin (Kultzer & Co., Wehrhein, Germany) and, after curing, samples were sectioned using a metallographical cutter (Isomet 1000; Buehler, Germany). The disc samples were polished using an abrasive paper sequence (Metaserv 3000; Buehler, Germany) to a ∼30-µm thickness and analyzed using light microscopy (Nikon E200, Japan). The bone growth was measured with respect to the implant platform at the bone contact with the healing abutment, according to the scheme using Image Tool software, version 5.02 for Microsoft Windows™. Two different investigators made the measurements at different times and a unique average of these values was computed. When the measured values were very different both investigators repeated measures.

Obtained results are shown in FIG. 5, 6 and 7 corresponding to groups A, B and C respectively.

In addition, histological analysis of the samples to measure the Bone-to-Implant Contact (hereinafter, BIC) was performed. Results are summarized in Table 3.

**Table 3. Results of BIC for the three groups of the study at 15, 30, 45 and 60 days.**

| Time of measurement | GROUP A BIC in % (mean ± SD) | GROUP B BIC in % (mean ± SD) | GROUP C BIC in % (mean ± SD) |
|---|---|---|---|
| 15 Days | 53.8 ± 2.3 | 61.7 ± 1.1 | 68.92 ± 0.3 |
| 30 Days | 56.24 ± 1.8 | 67.4 ± 1.8 | 69.35 ± 2.2 |
| 45 Days | 60.45 ± 1.2 | 68.1 ± 1.6 | 70.34 ± 1.1 |
| 60 Days | 68.29 ± 0.8 | 71.39 ± 1.1 | 73.89 ± 1.9 |

As can be seen in FIGs. 5 to 7, the implants were in contact with predominantly cortical bone along the upper threads in the cortical region, while the threads in the bone marrow were in contact with either newly formed bone or with normal bone marrow. A demarcation line was consistently seen between the newly formed bone and the old bone tissue. In general terms, from said figures it can be seen that an earlier formation of bone is induced in Groups B and C (even earlier and greater in the latter). This difference was maintained all throughout the experimental time frame (i.e., until day 60).

In addition, from table 3 it is also derivable an earlier and increased BIC in groups B and C compared to group A. In addition, and surprisingly, as also seen in FIGs 5 to 7, group C showed also an increase in all time points when compared with group B.

Finally, the quantity of new bone formed was measured also at 15, 30, 45 and 60 days.

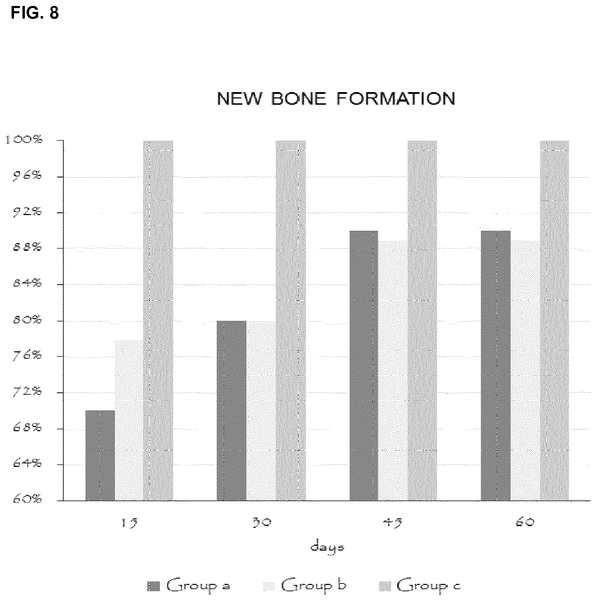

Results are summarized in FIG. 8 and are consistent with what has been discussed above. It can be seen an earlier formation of bone in groups B and C (group C shows an even higher bone formation in day 15 when compared with group B). In addition, group C is the only group that surprisingly showed an increase in new bone formed on day 60 (on said day, the quantity of bone formed in groups A and B had evolved to be similar).

### Example 4. Comparison between treatments

8 g of NaCl, 0.2 g of KCI, 1.44 g of Na₂HPO₄, 0.24 g of KH₂PO₄, 0.80 g of MgCl₂, 1.20 g of CaCl₂ and 20 g of EDTA were dissolved in 800 ml of water.

To analyze relative osteogenic capacity of PBS, CaCl₂, MgCl₂ and EDTA, DPPSC (1x10³ cells per cm²) were seeded in titanium discs of Ti₆Al₄V.
Group 1: treated with PBS (8 g of NaCl, 0.2 g of KCI, 1.44 g of Na₂HPO₄, 0.24 g of KH₂PO₄, dissolved in 800 ml of water).
Group 2: treated with PBS+CaCl₂+MgCl₂ (8 g of NaCl, 0.2 g of KCI, 1.44 g of Na₂HPO₄, 0.24 g of KH₂PO₄, 0.80 g of MgCl₂, 1.20 g of CaCl₂ dissolved in 800 ml of water).
Group 3: treated with PBS+CaCl₂+MgCl₂+EDTA (8 g of NaCl, 0.2 g of KCI, 1.44 g of Na₂HPO₄, 0.24 g of KH₂PO₄, 0.80 g of MgCl₂, 1.20 g of CaCl₂ and 20 g of EDTA were dissolved in 800 ml of water):
Group 4: treated with PBS+EDTA (8 g of NaCl, 0.2 g of KCI, 1.44 g of Na₂HPO₄, 0.24 g of KH₂PO₄ and 20 g of EDTA were dissolved in 800 ml of water).
Control: No treated discs.

Discs were treated and incubated during one hour before seeding cells in 24-well plates at a density of 1x10³ cells per cm². RT-PCR was performed at day 7 of osteoblast differentiation using OC, OCN and ALP. The differences in expression shown in FIG. 9 show that titanium discs treated with PBS with the addition of CaCl₂ and MgCl₂ presents more osteogenic capacity in comparison with treatment with only PBS or PBS+EDTA and that the combination of PBS, CaCl₂, MgCl₂ and EDTA shows and even more surprising osteogenic capacity compared to the other treatments.

Values of FIG. 9 are expressed as expression related to no treated discs at day 7.

Two commercial PBS were also tested and the results were similar to those of the PBS used in the above-mentioned Group 1. Said commercial PBS were: Sigma Aldrich P3813 SIGMA - Phosphate buffered saline (Contents of one pouch, when dissolved in one liter of distilled or deionized water, will yield 0.01 M phosphate buffered saline (NaCl 0.138 M; KCI - 0.0027 M); pH 7.4, at 25 °C).

Thermo Fisher CatNum 18912 - PBS Tablets. (Phosphate (as sodium phosphates) 10.0 mM, Potassium Chloride (KCI) 2.68 mM, Sodium Chloride (NaCl) 140.0 mM, to be dissolved with 500 ml distilled water).

### Example 5. Application of the composition

Application of the composition in a patient when using an implant: For implants placement using the bone bioactive composition, the drilling sequence bone has to according to the manufacturer's recommendations subsequently implant placing and with very low insertion between 20 to 50 Newtons. This is applicable for dental implants as well as for traumatology implants.

Application of the composition in a patient with periodontitis or peri-implantitis: For the periodontitis treatment using the bone bioactive composition, first of all the conventional periodontal treatment should be done; removing bacterial plaque and granulation tissue by supragingival and subgingival scrapings. In the same intervention, the area of the bone defect has to be well dry before applying the bone bioactive composition using a conventional brush; after that, bleeding is caused until the clot is formed. This process should be done more than once until recuperate all lost bone.

### BIBLIOGRAPHIC REFERENCES

### Non-patent literature

Albrektsson T, et al. "Osseointegrated titanium implants Requirements for ensuring a long-lasting, direct bone-to-implant anchorage in man" Acta Orthop Scand 1981,52:155-170
Anselme K "Osteoblast adhesion on biomaterials" Biomaterials 2000,21:667
García AJ "Get a grip: integrins in cell-biomaterial interactions" Biomaterials 2005,26:7525
Howlett CR, et al. "Mechanism of initial attachment of cells derived from human bone to commonly used prosthetic materials during cell culture" Biomaterials 1994, 15:213
Kilpadi KL, et al. "Hydroxylapatite binds more serum proteins, purified integrins, and osteoblast precursor cells than titanium or steel" J Biomed Mater Res 2001,57:258
Mohan S, et al. "Bone growth factors" Clin Orthop Relat Res 1991,263:30-48
Scotchford CA, et al. "Chemically patterned, metal-oxide-based surfaces produced by photolithographic techniques for studying protein- and cell-interactions. II: Protein adsorption and early cell interactions" Biomaterials 2003,24:1147
Steele JG, et al. "Attachment of human bone cells to tissue culture polystyrene and to unmodified polystyrene: the effect of surface chemistry upon initial cell attachment" J Biomater Sci Polym Ed 1993,5:245
Urist MR, et al. "Bone cell differentiation and growth factors" Science 1983,220:680-686
Wozney JM, et al. "Growth factors influencing bone development" J Cell Sci Suppl 1990,13:149-156
Yang J et al. "Effects of oestrogen deficiency on rat mandibular and tibial microarchitecture" Dentomaxillofac Radiol 2003 Jul,32(4):247-51

## Claims

1. Bone bioactive composition comprising a water-based salt solution comprising disodium hydrogen phosphate and sodium chloride, for use in promoting osteogenesis.

2. Bone bioactive composition for use according to claim 1, wherein disodium hydrogen phosphate is in a concentration from 8 to 12 mM in the solution and sodium chloride is in a concentration from 130 to 140 mM in the solution.

3. Bone bioactive composition for use according to any of claims 1-2, wherein the water-based salt solution further comprises potassium hydrogen phosphate and potassium chloride.

4. Bone bioactive composition for use according to any of claims 1-3, wherein potassium hydrogen phosphate is in a concentration from 1.4 to 2.2 mM in the solution, and potassium chloride is in a concentration from 2.3 to 3.1 mM in the solution.

5. Bone bioactive composition for use according to any of claims 1-4, wherein the pH of the solution is between 7.4 and 7.8.

6. Bone bioactive composition for use according to claim 5, wherein the pH of the solution is 7.6.

7. Bone bioactive composition for use according to any of claims 1-6, wherein the solution further comprises magnesium chloride and calcium chloride.

8. Bone bioactive composition for use according to any of claims 1-7, wherein magnesium chloride is in a concentration from 2 to 12 mM in the solution and calcium chloride is in a concentration from 7 to 15 mM in the solution.

9. Bone bioactive composition for use according to any of claims 1-8, wherein the solution further comprises a chelating agent.

10. Bone bioactive composition for use according to claim 9, wherein the chelating agent is EDTA.

11. Bone bioactive composition for use according to any of the claims 1-10, wherein the elements of the composition are in solid form.

12. Bone bioactive composition for use according to any of claims 1-11, for use in promoting osteogenesis when a metal implant is used.

13. Bone bioactive composition for use according to claim 12, wherein the metal implant is a dental titanium implant.

14. Bone bioactive composition for use according to any of the claims 1-13, for use in promoting osteogenesis in the treatment of a periodontal disease.

15. Bone bioactive composition comprising a water-based salt solution comprising 10 mM sodium dihydrogen phosphate, 137 mM sodium chloride, 1.8 mM potassium hydrogen phosphate, 2.7 mM potassium chloride, 8.4 mM magnesium chloride, 10.81 mM calcium chloride, and 68.4 mM EDTA.

16. Bone bioactive composition according to claim 15, wherein the pH of the solution is 7.6.


SEQUENCE LISTING

<110>  UNIVERSITAT INTERNACIONAL DE CATALUNYA,FUNDACIî PRIVADA
       UNIVERSIDAD CATîLICA DE MURCIA PD10139EP00 SEQUENCE LISTING

<120>  Bone bioactive composition and uses thereof

<130>  PD10139EP00

<160>  24

<170>  PatentIn version 3.5

<210>  1
<211>  20
<212>  DNA
<213>  Artificial Sequence

<220>
<223>  Forward primer ALP_1

<400>  1
ggacatgcag tacgagctga                                                   20


<210>  2
<211>  20
<212>  DNA
<213>  Artificial Sequence

<220>
<223>  Reverse primer ALP_1

<400>  2
gtcaattctg cctccttcca                                                   20


<210>  3
<211>  21
<212>  DNA
<213>  Artificial Sequence

<220>
<223>  Forward primer ALP_2

<400>  3
ccgtggcaac tctatctttg g                                                 21


<210>  4
<211>  20
<212>  DNA
<213>  Artificial Sequence

<220>
<223>  Reverse primer ALP_2

<400>  4
gccatacagg atggcagtga                                                   20


<210>  5
<211>  20

<212>  DNA
<213>  Artificial Sequence

<220>
<223>  Forward primer COL1_1

<400>  5
actggtgaga cctgcgtgta                                                    20


<210>  6
<211>  19
<212>  DNA
<213>  Artificial Sequence

<220>
<223>  Reverse primer COL1_1

<400>  6
cagtctgctg gtccatgta                                                     19


<210>  7
<211>  22
<212>  DNA
<213>  Artificial Sequence

<220>
<223>  Forward primer COL1_2

<400>  7
ccctggaaag aatggagatg at                                                 22


<210>  8
<211>  23
<212>  DNA
<213>  Artificial Sequence

<220>
<223>  Reverse primer COL1_2

<400>  8
actgaaacct ctgtgtccct tca                                                23


<210>  9
<211>  19
<212>  DNA
<213>  Artificial Sequence

<220>
<223>  Forward primer OC_1

<400>  9
gtgcagcctt tgtgtccaa                                                     19


<210>  10
<211>  19
<212>  DNA
<213>  Artificial Sequence

<220>
<223>  Reverse primer OC_1

<400>  10
gctcacacac ctccctcct                                                    19


<210>  11
<211>  20
<212>  DNA
<213>  Artificial Sequence

<220>
<223>  Forward primer OC_2

<400>  11
aagagaccca ggcgctacct                                                   20


<210>  12
<211>  21
<212>  DNA
<213>  Artificial Sequence

<220>
<223>  Reverse primer OC_2

<400>  12
aactcgtcac agtccggatt g                                                 21


<210>  13
<211>  20
<212>  DNA
<213>  Artificial Sequence

<220>
<223>  Forward primer RUNX2_1

<400>  13
ttactgtcat ggcgggtaac                                                   20


<210>  14
<211>  19
<212>  DNA
<213>  Artificial Sequence

<220>
<223>  Reverse primer RUNX"_1

<400>  14
ggttcccgag gtccatcta                                                    19


<210>  15
<211>  23
<212>  DNA
<213>  Artificial Sequence

<220>
<223>  Forward primer RUNX2_2

<400>  15
agcaaggttc aacgatctga gat                                              23


<210>  16
<211>  23
<212>  DNA
<213>  Artificial Sequence

<220>
<223>  Reverse primer RUNX2_2

<400>  16
tttgtgaaga cggttatggt caa                                              23


<210>  17
<211>  20
<212>  DNA
<213>  Artificial Sequence

<220>
<223>  Forward primer ITGa4

<400>  17
tccgagtcaa tgtccacaga                                                  20


<210>  18
<211>  20
<212>  DNA
<213>  Artificial Sequence

<220>
<223>  Reverse primer ITGa4

<400>  18
gctgggctac cctattcctc                                                  20


<210>  19
<211>  25
<212>  DNA
<213>  Artificial Sequence

<220>
<223>  Forward primer GAPDH

<400>  19
ctggtaaagt ggatattgtt gccat                                            25


<210>  20
<211>  26
<212>  DNA
<213>  Artificial Sequence

<220>
<223>  Reverse primer GAPDH

<400>  20
tggaatcata ttggaacatg taaacc                                           26

<210>  21
<211>  20
<212>  DNA
<213>  Artificial Sequence

<220>
<223>  Forward primer BMP2

<400>  21
gcggaaacgc cttaagtcca                                                    20


<210>  22
<211>  20
<212>  DNA
<213>  Artificial Sequence

<220>
<223>  Reverse primer BMP2

<400>  22
gtggagttca gatgatcagc                                                    20


<210>  23
<211>  20
<212>  DNA
<213>  Artificial Sequence

<220>
<223>  Forward primer OCN

<400>  23
gcagacctga catccagtac                                                    20


<210>  24
<211>  20
<212>  DNA
<213>  Artificial Sequence

<220>
<223>  Reverse primer OCN

<400>  24
taatctggac tgcttgtggc                                                    20